# FASCICULE DE BREVET EUROPEEN

(11) **EP 0 487 713 B1**
(45) Date de publication et mention de la délivrance du brevet: **21.08.1996**
(21) Numéro de dépôt: 91912709.2
(22) Date de dépôt: 13.06.1991
(51) Int. Cl.: A61L 25/00

(54) **COLLE BIOLOGIQUE FLUIDE**
FLUSSIGER BIOLOGISCHER KLEBER
FLUID BIOLOGICAL GLUE

(30) Priorité: 15.06.1990 FR 9007505
(43) Date de publication de la demande: 03.06.1992
(73) Titulaire: FONDATION NATIONALE DE TRANSFUSION SANGUINE, F-75739 Paris Cédex 15 (FR)
(72) Inventeur: CHABBAT, Jacques, F-75017 Paris (FR); COURTEILLE, Frédéric, F-94230 Cachan (FR)
(74) Mandataire: Ahner, Francis
(86) Numéro de dépôt international: FR9100475
(87) Numéro de publication internationale: WO9119519

(56) Documents cités:
- EP-A- 0 085 923

## Description

La présente invention concerne une colle biologique pour tissus humains ou animaux, stabilisée sous une forme fluide à basse température.

Le type de colle utilisée pour réunir des tissus humains ou animaux est en général un concentré de facteurs de l'hémostase, coagulable par la thrombine, et riche en fibrinogène.

Ces colles sont en fait composées de deux constituants essentiels, destinés à être mélangés extemporanément lors de leur application. Le premier composant contient essentiellement du fibrinogène. Il est le plus souvent constitué d'un mélange de protéines extraites du plasma humain et contient, en plus du fibrinogène, de la fibronectine et du facteur XIII. Quant au second composant, il contient essentiellement de la thrombine calcique, qui peut être d'origine humaine ou animale, bovine par exemple. Ce type de colle est notamment décrit dans les brevets EP 103 196, EP 253 198, EP 37 078 et EP 183 674.

La demande EP- 85 923 décrit des préparations de fibrinogène lyophilisé contenant de l'arginine.

Très récemment, la Demanderesse a proposé une nouvelle colle présentant l'avantage d'être sous une forme pasteurisée, (FR 89 10355, déposé le 1.8.89 au nom de la Fondation Nationale de Transfusion Sanguine). Cette colle pasteurisée permet ainsi de s'affranchir de tout risque de contamination virale, pouvant provenir du fibrinogène extrait du plasma humain.

L'un des inconvénients de ces colles biologiques est leur instabilité au stockage. En effet, si l'on conserve la colle à la température de 4°C, la thrombine demeure stable mais le fibrinogène tend à polymériser; par contre, à une température de l'ordre de 20 à 25°C, c'est la thrombine qui se détériore. C'est pourquoi, l'une des techniques utilisées pour conserver ces colles consiste à les congeler. Evidemment, lors de l'utilisation de ces colles, il est alors nécessaire de les décongeler, ce qui est mal commode, et en outre prend du temps, car il n'est pas possible de les chauffer brutalement au risque d'en dégrader les composants.

Faute d'utiliser ce type de colle, il est alors nécessaire d'employer des colles préparées depuis peu de temps. Il est évident que la préparation de telles colles pour ainsi dire au jour le jour rend la gestion industrielle de ce type de produit très difficile.

Il était donc intéressant de mettre au point une colle utile à la consommation, sous forme liquide, qui soit ainsi directement prête à l'emploi.

C'est précisément l'objet de la présente invention de proposer une colle utile à la consommation sous forme fluide, qui puisse être stockée à basse température, c'est-à-dire entre +2 et +10°C, de préférence, comme cela est habituel pour les produits de ce type, au voisinage de 4°C, la température des réfrigérateurs.

Pour ce faire, la présente invention se rapporte à une colle biologique pour tissus humains ou animaux du type à deux composants, comportant :
- un premier composant à base de fibrinogène, et
- un second composant à base de thrombine,
destinés à être mélangés extemporanément au moment de l'emploi, caractérisée en ce que le composant à base de fibrinogène contient au moins un agent chaotrope, et en ce que la colle est liquide à basse température.

En effet, il a été mis en évidence que l'adjonction d'un agent chaotrope, et en particulier l'urée, permettait de prévenir la polymérisation du fibrinogène à basse température. Il devient alors possible de conserver les deux composants de la colle biologique, sous une forme fluide, a une même température. Ceci présente un net avantage tant pour le stockage que sur le plan commodité pour les utilisateurs, qui disposent immédiatement d'une colle sous forme fluide.

L'invention peut être mise en oeuvre avec une colle biologique quelconque du type à deux composants. Elle est plus particulièrement intéressante avec la colle objet du brevet FR 89 10355 précédemment identifié.

Le composant à base de fibrinogène, utilisé dans ce type de colle, provient la plupart du temps d'un mélange de protéines extraites de plasma humain, comme cela a été mentionné précédemment. Il est donc nécessaire de lui faire subir une inactivation virale. Cette inactivation peut ainsi être réalisée par pasteurisation dudit composant.

Quant au composant à base de thrombine, il s'agit surtout de thrombine calcique.

Ces deux composants sont stockés, soit séparément dans des flaconnages différents, soit dans des dispositifs applicateurs qui assurent leur mélange lors de l'application, notamment des dispositifs à deux seringues jumelées.

Quel que soit le type de colle ou de dispositif, la présente invention présente un intérêt considérable.

L'agent chaotrope est utilisé à des concentrations comprises de préférence entre 0,3 et 1 M, notamment à une concentration proche de 0,5 M.

Il faut en outre tenir compte du fait que l'ajout de cet agent chaotrope est limité pour des raisons d'osmolarité, laquelle doit demeurer compatible avec le milieu où la colle doit être appliquée.

C'est pourquoi, selon un mode de réalisation particulier de l'invention, ce composant à base de fibrinogène subit une étape de réduction de son osmolarité, notamment avant l'ajout de l'urée. L'osmolarité du composant à base de fibrinogène est initialement comprise habituellement entre 700 et 800 mosmol. Il est clair que l'ajout durée à celui-ci implique une augmentation de celle-ci. Une valeur trop importante de cette osmolarité pouvant entraîner un risque de toxicité cellulaire, il est par conséquent prudent de réduire l'osmolarité initiale, de manière à ce qu'elle demeure compatible avec les conditions physiologiques en présence d'urée.

Cette réduction de l'osmolarité peut être ainsi réalisée en effectuant une dialyse par ultrafiltration du composant à base de fibrinogène, ou par tout autre moyen.

Enfin, il est possible d'améliorer la qualité d'adhésivité de la colle biologique selon l'invention en ajoutant au composant à base de fibrinogène et stabilisé avec de l'urée au moins un agent adhésif. Il peut ainsi s'agir de collagène, de glycérol ou encore d'un acide aminé. A titre d'acide aminé on peut en particulier citer la lysine, la glycine ou l'acide glutamique.

Les exemples donnés ci-après permettront de mettre en évidence d'autres avantages et caractéristiques de la présente invention, sans pour autant en limiter la portée.

### PREPARATION DU COMPOSANT A BASE DE FIBRINOGENE

A partir de 280 l de plasma, la cryoprécipitation a permis de récupérer 2 kg de précipité (cryoprécipité). Les protéines entrant dans la composition de la colle sont obtenues après remise en solution de ce précipité (1 kg/4 litres) dans un tampon tris 20 mM pH 7, suivi d'une précipitation à la température de 25°C après addition d'héparine à 32 U/ml. La suspension ainsi obtenue est centrifugée, le culot (environ 1 kg) est récupéré puis remis en solution dans du citrate trisodique 70 mM - glycine 0,1 M - NaCl 0,03 M - acide aminocaproïque 50 mM - arginine 50 mM et aprotinine 100 U/ml à pH 8 (1 kg/1 litre). On procède alors à un chauffage liquide 10 heures à 60°C après avoir ajouté des stabilisants de protéines, à savoir 2400 g de glucose et 600 g de sorbitol à 2 litres de solution pour un volume final d'environ 4 litres (compte-tenu de la dilution due à l'addition des sucres). Après pasteurisation, la solution est diluée 10 fois dans du citrate 5 mM-NaCl 0,051 M - acide -aminocaproïque 25 mM - arginine 17 mM pH 7. La solution obtenue est alors précipitée à l'alcool à 10% final à une température comprise entre -2 et -3°C. Les protéines précipitées sont récupérées dans le culot de centrifugation. Celui-ci est ensuite remis en solution dans du citrate trisodique 1 mM - NaCl 60 mM - acide -aminocaproïque 20 mM - glycine 60 mM pH 7,5.

Les protéines sont ensuite concentrées jusqu'à un taux de 27 - 30 g/l pour un volume final de 1,5 l auquel on rajoute du polysorbate à 0.25‰ et du caprylate de sodium à 0,15 g/l. Le produit est ensuite réparti en flacons après filtration stérilisante puis lyophilisé. La concentration en protéines coagulables peut être modulée en fonction du volume de reconstitution du lyophilisat. Le produit obtenu présente, pour un taux de protéines de (85-150) g/l, (80-120) g/l de fibrinogène coagulable, (4-15) g/l de fribronectine et un taux d'aprotinine de 2 000 KIU/ml (solvant utilisé pour la reconstitution du lyophilisat).

### EXEMPLE I

Trois solutions de fibrinogène à 110 g/l, obtenues selon le mode opératoire décrit précédemment, sont additionnées d'urée à différentes concentrations (U1, U2 et U3), et testées pour leur stabilité dans le temps à la température de 4°C. La stabilité est évaluée par la concentration de fibrinogène coagulable et par l'adhésivité de la colle après ajout de la thrombine calcique, après un mois.

Les résultats obtenus sont présentés dans le tableau I ci-après. Ce tableau rend également compte à titre comparatif des résultats observés avec un témoin à base de fibrinogène, c'est-à-dire sans urée.

On constate que l'adhésivité des compositions varie de manière importante entre le témoin et la colle contenant 0,5 M d'urée.

**TABLEAU I**

| | FIBRINOGENE COAGULABLE (g/l) | | | Adhésivité TO g/cm² | Adhésivité 1 mois à 4°C g/cm² |
|---|---|---|---|---|---|
| | 1 sem 4°C | 3 sem 4°C | 5 sem 4°C | | |
| Témoin | 88 | 99 | 114,5 | 100 | 59,1 |
| U1 (0,15M) | 89,5 | 104,5 | 107,5 | 100 | 68,7 |
| U2 (0,25M) | 93,5 | 102,5 | 110,5 | 100 | 63 |
| U3 (0,50M) | 91,5 | 99 | 106 | 100 | 100,7 |
| N.B. : Les échantillons Témoin, U1 et U2 ont été préalablement fluidifiés à 37°C pour les différentes analyses. | | | | | |

### EXEMPLE II

Les quatre solutions à base de fibrinogène décrites dans l'exemple I ont en outre subi des tests complémentaires afin de déterminer leur pH, leur osmolarité, et d'apprécier leur durée de fluidification pour passer d'une température de 4°C à la température ambiante.

Les résultats figurent dans le tableau II.

**TABLEAU II**

| | Etat à 4°C | Temps de rétablissement de la fluidité à 20°C | pH | Osmolarité mOsm/l |
|---|---|---|---|---|
| Témoin | Gélifié | Préchauffage à 37°C: non fluide | 7,4 | 787 |
| U1 | Visqueux | 30 min. | 7,38 | 912 |
| U2 | Visqueux | 15 min. | 7,51 | 1 020 |
| U3 | Fluide | Fluide | 7,48 | 1 184 |

### EXEMPLE III

Cet essai a été réalisé en utilisant une solution de fibrinogène à 110 g/l, obtenue selon le mode opératoire décrit précédemment, hormis l'étape de concentration-dialyse qui a été réalisée par ultra-filtration sur cassette 100 K.

Une partie de la solution finale obtenue a été stockée après addition d'urée qsp 0,5 M, à deux ambiances correspondant respectivement aux températures de +4°C et de +25°C.

Cet essai montre que lorsque l'osmolarité est contenue dans des normes physiologiques du fait de la concentration par ultra-filtration, le produit reste fluide et stable en présence d'urée 0,5 M, après un mois de stockage a °4°C et à +25°C. On ne constate aucune altération des caractéristiques physico-chimiques et en particulier de l'adhésivité, par suite de l'abaissement d'osmolarité consécutif à la dialyse par ultra-filtration.

Les résultats sont présentés dans le tableau III ci-après.

**TABLEAU III**

| | Témoin TO sans urée | Témoin TO + urée 0,5M | + urée 0,5M 1 mois à 4°C | + urée 0,5M 1 mois à 25°C |
|---|---|---|---|---|
| PROTEINES g/l | 126 | 113 | 111 | 111,5 |
| Fg COAG. g/l | 100 | 105 | 96,5 | 93 |
| FIBRONECTINE g/l | 6,1 | 7,1 | --- | --- |
| pH | 7,6 | 7,5 | 7,5 | 7,4 |
| OSMOLARITE mosmol/l | 208 | 700 | 723 | 718 |
| ADHESIVITE g/cm² | 139 | 140 | 145 | 141 |

## Revendications (Revendications pour l'(les) Etat(s) contractant(s) suivant(s): AT, BE, CH, DE, DK, FR, GB, IT, LI, LU, NL, SE)

1. Colle biologique non lyophilisée, pour tissus humains ou animaux, du type à deux composants, comportant :
- un premier composant à base de fibrinogène, et
- un second composant à base de thrombine
destinés à être mélangés extemporanément au moment de l'emploi, caractérisée en ce que le composant à base de fibrinogène contient au moins un agent chaotrope à une concentration comprise entre environ 0,3M et 1M, et en ce que la colle est liquide à température de stockage.

2. Colle biologique selon la revendication 1, caractérisée en ce que l'agent chaotrope est l'urée.

3. Colle biologique selon l'une des revendications 1 ou 2, caractérisée en ce que l'urée est présente dans le composant à base de fibrinogène à une concentration proche de 0,5M.

4. Colle biologique selon l'une des revendications 1 à 3, caractérisée en ce que le composant à base de fibrinogène a subi au préalable une inactivation virale.

5. Colle biologique selon l'une des revendications 1 à 4, caractérisée en ce que l'osmolarité du composant à base de fibrinogène a été réduite avant l'addition de l'agent chaotrope.

6. Colle biologique selon la revendication 5, caractérisée en ce que la réduction de l'osmolarité a été réalisée en effectuant une dialyse par ultrafiltration du composant à base de fibrinogène.

7. Colle biologique selon l'une des revendications 1 à 6, caractérisée en ce que le composant à base de fibrinogène contient en outre au moins un composé favorisant l'adhésivité de la colle.

8. Colle biologique selon la revendication 7, caractérisée en ce que le composé favorisant l'adhésivité peut être choisi parmi le collagène, le glycérol et un acide aminé, dont en particulier la lysine, la glycine et l'acide glutamique.

## Revendications (Revendications pour l'(les) Etat(s) contractant(s) suivant(s): ES, GR)

1. Procédé de préparation d'une colle biologique non lyophilisée, pour tissus humains ou animaux, caractérisé en ce qu'on prépare séparément :
- un premier composant à base de fibrinogène contenant au moins un agent chaotrope à une concentration comprise entre environ 0,3M et 1M,
- un second composant à base de thrombine,
ces deux composants étant liquides à température de stockage et destinés à être mélangés extemporanément au moment de l'emploi.

2. Procédé selon la revendication 1, caractérisé en ce que l'agent chaotrope est l'urée.

3. Procédé selon l'une des revendications 1 ou 2, caractérisé en ce que l'urée est présente dans le composant à base de fibrinogène à une concentration proche de 0,5M.

4. Procédé selon l'une des revendications 1 à 3, caractérisé en ce que le composant à base de fibrinogène a subi au préalable une inactivation virale.

5. Procédé selon l'une des revendications 1 à 4, caractérisé en ce que l'osmolarité du composant à base de fibrinogène a été réduite avant l'addition de l'agent chaotrope.

6. Procédé selon la revendication 5, caractérisé en ce que la réduction de l'osmolarité a été réalisée en effectuant une dialyse par ultrafiltration du composant à base de fibrinogène.

7. Procédé selon l'une des revendications 1 à 6, caractérisé en ce que le composant à base de fibrinogène contient en outre au moins un composé favorisant l'adhésivité de la colle.

8. Procédé selon la revendication 7, caractérisé en ce que le composé favorisant l'adhésivité peut être choisi parmi le collagène, le glycérol et un acide aminé, dont en particulier la lysine, la glycine et l'acide glutamique.

## Claims (Claims for the following Contracting State(s): AT, BE, CH, DE, DK, FR, GB, IT, LI, LU, NL, SE)

1. Two-part nonfreeze-dried biological glue for human or animal tissue comprising:
- a fibrinogen-based first component, and
- a thrombin-based second component
intended to be mixed freshly at the time of use, characterized in that the fibrinogen-based component contains at least one chaotropic agent at a concentration of between about 0.3 M and 1 M, and in that the glue is liquid at storage temperature.

2. Biological glue according to Claim 1, characterized in that the chaotropic agent is urea.

3. Biological glue according to either of Claims 1 and 2, characterized in that the urea is present in the fibrinogen-based component at a concentration close to 0.5 M.

4. Biological glue according to one of Claims 1 to 3, characterized in that the fibrinogen-based component was previously subjected to a viral inactivation.

5. Biological glue according to one of Claims 1 to 4, characterized in that the osmolarity of the fibrinogen-based component was reduced before the addition of the chaotropic agent.

6. Biological glue according to Claim 5, characterized in that the reduction in osmolarity was achieved by carrying out a dialysis by ultrafiltration of the fibrinogen-based component.

7. Biological glue according to one of Claims 1 to 6, characterized in that the fibrinogen-based component contains, in addition, at least one component enhancing the adhesiveness of the glue.

8. Biological glue according to Claim 7, characterized in that the compound enhancing adhesiveness may be chosen from collagen, glycerol and an amino acid, including in particular lysine, glycine and glutamic acid.

## Claims (Claims for the following Contracting State(s): ES, GR)

1. Process for the preparation of a nonfreeze-dried biological glue for human or animal tissue, characterized in that there are prepared separately:
- a fibrinogen-based first component containing at least one chaotropic agent at a concentration of between about 0.3 M and 1 M,
- a thrombin-based second component,
these two components being liquid at the storage temperature and intended to be mixed freshly at the time of use.

2. Process according to Claim 1, characterized in that the chaotropic agent is urea.

3. Process according to either of Claims 1 and 2, characterized in that the urea is present in the fibrinogen-based component at a concentration close to 0.5 M.

4. Process according to one of Claims 1 to 3, characterized in that the fibrinogen-based component was previously subjected to a viral inactivation.

5. Process according to one of Claims 1 to 4, characterized in that the osmolarity of the fibrinogen-based component was reduced before the addition of the chaotropic agent.

6. Process according to Claim 5, characterized in that the reduction in osmolarity was achieved by carrying out a dialysis by ultrafiltration of the fibrinogen-based component.

7. Process according to one of Claims 1 to 6, characterized in that the fibrinogen-based component contains, in addition, at least one component enhancing the adhesiveness of the glue.

8. Process according to Claim 7, characterized in that the compound enhancing adhesiveness may be chosen from collagen, glycerol and an amino acid, including in particular lysine, glycine and glutamic acid.

## Patentansprüche (Patentansprüche für folgende(n) Vertragsstaat(en): AT, BE, CH, DE, DK, FR, GB, IT, LI, LU, NL, SE)

1. Nicht lyophilisierter biologischer Kleber für menschliche oder tierische Gewebe vom 2-Komponenten-Typ, umfassend:
- einen ersten Bestandteil auf Fibrinogenbasis und
- einen zweiten Bestandteil auf Thrombinbasis,
die dazu bestimmt sind, unmittelbar vor der Verwendung gemischt zu werden, dadurch charakterisiert, daß der Bestandteil auf Fibrinogenbasis mindestens ein chaotropes Mittel in einer Konzentration zwischen etwa 0,3M und 1M enthält, und daß der Kleber bei der Lagertemperatur flüssig ist.

2. Biologischer Kleber nach Anspruch 1, dadurch charakterisiert, daß das chaotrope Mittel Harnstoff ist.

3. Biologischer Kleber nach einem der Ansprüche 1 oder 2, dadurch charakterisiert, daß der Harnstoff im Bestandteil auf Fibrinogenbasis in einer Konzentration von ungefähr 0,5M vorhanden ist.

4. Biologischer Kleber nach einem der Ansprüche 1 bis 3, dadurch charakterisiert, daß der Bestandteil auf Fibrinogenbasis zuvor einer Virusinaktivierung unterworfen wurde.

5. Biologischer Kleber nach einem der Ansprüche 1 bis 4, dadurch charakterisiert, daß die Osmolarität des Bestandteils auf Fibrinogenbasis vor Zugabe des chaotropen Mittels reduziert wurde.

6. Biologischer Kleber nach Anspruch 5, dadurch charakterisiert, daß die Verminderung der Osmolarität des Bestandteils auf Fibrinogenbasis durch Durchführung einer Dialyse durch Ultrafiltration bewirkt wurde.

7. Biologischer Kleber nach einem der Ansprüche 1 bis 6, dadurch charakterisiert, daß der Bestandteil auf Fibrinogenbasis zusätzlich mindestens eine die Klebefähigkeit des Klebers begünstigende Verbindung enthält.

8. Biologischer Kleber nach Anspruch 7, dadurch charakterisiert, daß die die Klebefähigkeit begünstigende Verbindung ausgewählt werden kann aus Collagen, Glycerin und einer Aminosäure und davon insbesondere Lysin, Glycin und Glutaminsäure.

## Patentansprüche (Patentansprüche für folgende(n) Vertragsstaat(en): ES, GR)

1. Verfahren zur Herstellung eines nicht lyophilisierten biologischen Klebers für menschliche oder tierische Gewebe, dadurch charakterisiert, daß man:
- einen ersten Bestandteil auf Fibrinogenbasis, der mindestens ein chaotropes Mittel in einer Konzentration zwischen etwa 0,3M und 1M enthält, und
- einen zweiten Bestandteil auf Thrombinbasis
getrennt herstellt, wobei die beiden Bestandteile bei der Lagertemperatur flüssig sind und dazu bestimmt sind, unmittelbar vor der Verwendung gemischt zu werden.

2. Verfahren nach Anspruch 1, dadurch charakterisiert, daß das chaotrope Mittel Harnstoff ist.

3. Verfahren nach einem der Ansprüche 1 oder 2, dadurch charakterisiert, daß der Harnstoff im Bestandteil auf Fibrinogenbasis in einer Konzentration von ungefähr 0,5M vorhanden ist.

4. Verfahren nach einem der Ansprüche 1 bis 3, dadurch charakterisiert, daß der Bestandteil auf Fibrinogenbasis zuvor einer Virusinaktivierung unterworfen wurde.

5. Verfahren nach einem der Ansprüche 1 bis 4, dadurch charakterisiert, daß die Osmolarität des Bestandteils auf Fibrinogenbasis vor Zugabe des chaotropen Mittels reduziert wurde.

6. Verfahren nach Anspruch 5, dadurch charakterisiert, daß die Verminderung der Osmolarität bewirkt wurde, indem eine Dialyse des Bestandteils auf Fibrinogenbasis durch Ultrafiltration durchgeführt wurde.

7. Verfahren nach einem der Ansprüche 1 bis 6, dadurch charakterisiert, daß der Bestandteil auf Fibrinogenbasis zusätzlich mindestens eine die Klebefähigkeit des Klebers begünstigende Verbindung enthält.

8. Verfahren nach Anspruch 7, dadurch charakterisiert, daß die die Klebefähigkeit begünstigende Verbindung ausgewählt werden kann aus Collagen, Glycerin und einer Aminosäure, und von diesen insbesondere Lysin, Glycin und Glutaminsäure.
